# EUROPEAN PATENT APPLICATION

(11) **EP 4 650 393 A1**
(43) Date of publication of application: **19.11.2025**
(21) Application number: 24741713.2
(22) Date of filing: 11.01.2024
(51) Int. Cl.: C08J 11/24, C08J 11/18

(54) **LOW-TEMPERATURE DEPOLYMERIZATION OF POLYMER CONTAINING URETHANE FUNCTIONAL GROUP USING COSOLVENT AND METHOD FOR PRODUCING POLYOL**

(30) Priority: 13.01.2023 KR 20230005562
(71) Applicant: Korea Research Institute of Chemical Technology, Daejeon 34114 (KR)
(72) Inventor: CHO, Joung Mo, Daejeon 34114 (KR); LE, Thi Hong Ngan, Daejeon 34114 (KR); JO, Ye Rim, Daejeon 34114 (KR)
(74) Representative: Isarpatent
(86) International application number: PCT/KR2024/000519
(87) International publication number: WO 2024/151092

(57) **Abstract**

The present invention relates to a low-temperature depolymerization of a polymer containing a urethane functional group using a co-solvent and to a method for producing a polyol. More specifically, the invention concerns adding a compound having two or more alcohol functional groups as a depolymerization solvent for decomposing a polymer containing a urethane functional group, and adding an aromatic compound having an alkoxy functional group as a co-solvent to construct a reaction system for decomposing the polymer containing a urethane functional group. This enables rapid depolymerization at a low temperature, and through a physical separation process of the reaction products generated therefrom, a high-quality regenerated polyol can be obtained at a high yield.

## Description

### [Technical Field]

The present invention relates to a composition for low-temperature depolymerization of a polymer containing a urethane functional group, such as waste polyurethane, a depolymerization method, and a method for producing polyol therefrom. More specifically, the invention relates to a method in which a compound having two or more alcohol functional groups is added as a depolymerization solvent for decomposing a polymer containing a urethane functional group, and an aromatic compound having one or more alkoxy functional groups is further added as a co-solvent, thereby forming a reaction system for decomposing the polymer containing a urethane functional group. This allows rapid depolymerization at a low temperature, and through a physical separation process of the reaction products thus obtained, high-quality recycled polyol can be obtained at a high yield.

### [Background Art]

Polyurethane is a polymer compound in which the main polymer chain is structured through urethane bonds, and is synthesized via a urethane polymerization reaction between a polyol having alcohol functional groups and an isocyanate compound having isocyanate functional groups at both ends. Polyurethane forms a polymer structure through a soft segment (SS), which is derived from the polyol and has a flexible structure and elastic recovery, and a hard segment (HS), which provides strong cohesion through the isocyanate and urethane bonds. Because various types of polyols and isocyanates can be used for synthesis, and the physical properties can be adjusted according to the blending conditions of the raw materials, polyurethane can be applied to a wide variety of materials.

Polyurethane can be classified into flexible foam, rigid foam, and semi-rigid foam according to its physical properties, and can be further divided into polyurethane foam (PUF)-manufactured into a lightweight honeycomb-like structure due to vigorous foaming generated during the manufacturing process or polymerization reaction-and non-foam urethane, which is mainly used in coatings (C), adhesives (A), sealants (S), and elastomers (E).

Due to the excellent physical properties and processability unique to these materials, polyurethane is widely used in general-purpose plastic products ranging from soft cushioning materials such as clothing, bed mattresses, and automobile seats, to thermal insulation materials, soundproofing materials, flooring, and various construction materials, including those applied in refrigerators, freezers, and heat-insulating pipes.

Polyurethane has been applied in a wide range of ways in modern daily life as various materials with different physical properties have been developed, and it is known that not only the scale of the related industry but also the amount of polyurethane waste has greatly increased. In particular, compared to other materials, polyurethane often has a shorter usage cycle, and its final form is often produced by cutting after synthesis rather than being directly molded through polymerization. As a result, polyurethane is one of the plastic products that generate a particularly large amount of process waste.

Especially, many types of polyurethane waste are in thermosetting forms that harden when heated, making them difficult to recycle by melting. Since there are few appropriate disposal methods, most are incinerated, and this process generates secondary pollutants such as harmful gases and dust, causing many environmental problems. Recently, as the severity of environmental issues caused by synthetic polymer waste has emerged, active research has been conducted on recycling technologies that can reduce the amount of waste plastics generated and minimize adverse effects on the environment. However, research on recycling technology for polyurethane has been relatively limited due to economic considerations, the quality of the final product and technical constraints.

Technologies available for recycling polyurethane include: physical recycling, in which the material is ground and processed into another form without altering its chemical structure; thermal recycling, in which the material is heated and converted into fuel form or the thermal energy is directly recovered; and chemical recycling, in which some or all of the material is recovered in the form of raw materials prior to material synthesis through chemical reactions.

The thermal recycling method can be used to obtain only energy through heating or to produce low-grade oil fractions by completely modifying the polymer structure via pyrolysis. However, since these products are consumed as fuel and cannot be regenerated repeatedly, this constitutes a non-environmentally friendly recycling method.

The physical recycling method is used in the form of reprocessing, and in most cases, it is applied to produce materials of lower grade or quality than before. Its applications and range of uses are very limited, and the number of recycling cycles is restricted; therefore, it cannot serve as a perfect regeneration method for overcoming environmental issues.

In contrast, the chemical recycling method can depolymerize polyurethane back into the raw materials prior to synthesis. This allows a portion of polyurethane, which was conventionally synthesized solely from petrochemical-derived raw materials, to be replaced with recycled raw materials. Accordingly, it is considered an environmentally friendly recycling method that enables the infinite and repeated use of resources.

The chemical recycling of polyurethane is carried out through a reaction in which waste polymers are brought into contact with a solvent and a catalyst, and heat is applied to break down the urethane bonds. Depending on the type of solvent used, the process can be classified into hydrolysis, glycolysis, acidolysis, or aminolysis. Among these chemical decomposition (or depolymerization) pathways, the glycolysis method is the most commonly applied depolymerization method, as the reaction proceeds well under relatively mild conditions and the purification process is relatively easy.

As prior art, U.S. Patent No. 4,243,560 (published January 6, 1981) discloses a technology in which flexible or semi-rigid polyurethane foam is heated to a high temperature in the absence of contact with oxygen, thereby decomposing the material and obtaining recyclable alcohols and polyols as distilled products. This technology is a pyrolysis method that does not use any additional additives or catalysts, and is characterized in that decomposition into products can be induced within a short residence time of several minutes to within an hour after the raw material is fed. However, since the depolymerization is performed at a high temperature of 450 °C to 600 °C, the process may involve excessive energy consumption, and a high investment cost is required for high-temperature reaction equipment. To overcome these problems, chemical depolymerization technologies have been developed in which a polyhydric alcohol solvent is added as a reactant to allow the polymer to be decomposed by breaking the urethane bonds followed by substitution of the alcohol functional groups, and a catalyst is used so that the depolymerization reaction can proceed at a temperature below the boiling point of the solvent.

For example, U.S. Patent No. 6,750,260 (published June 15, 2004) discloses a method in which glycolysis is performed at a temperature of 180 °C to 200 °C by using a polyhydric alcohol solvent such as diethylene glycol, as the reactant and adding KOH as the catalyst to carry out depolymerization. An epoxide-containing compound, such as propylene oxide or ethylene oxide, is then further added to the polyol intermediate obtained from the depolymerization product to produce recycled polyol. In glycolysis reactions where a polyhydric alcohol is used as the solvent to induce the decomposition of polyurethane, the breakdown of urethane bonds yields polyols and diamine compounds as reaction products. However, in some cases, side reactions occur due to contact with oxygen or oxygen-containing compounds, resulting in a dark, opaque, turbid black mixture as the product. Even if the depolymerization product thus obtained contains multiple active alcohol groups, there are limitations in re-synthesizing or recycling it into polyurethane materials of the same or similar quality.

U.S. Patent No. 6,020,386 (published February 1, 2000) discloses a glycolysis depolymerization method in which the addition of a dialkyl carbonate or a dicarbonyl compound can minimize the amount of diamine compounds generated in the depolymerization product.

Korean Registered Patent No. 10-0278099 (published January 15, 2001) discloses a method in which, after carrying out depolymerization of a polyurethane elastomer through a glycolysis reaction, a compound having polycarboxylic acid functional groups is added to the reaction product to induce an esterification reaction of the glycol used as the solvent thereby converting it into a high-molecular-weight compound, and the resulting product is used as a raw material for recycled polyurethane. In this technology, the reaction conditions are presented as performing glycolysis at a reaction temperature of 120 °C to 300 °C for 30 minutes to 15 hours. However, under low-temperature conditions, the depolymerization rate is very slow, resulting in very low product yield, whereas under high-temperature conditions, the depolymerization rate can be increased but side reactions may be promoted, making it difficult to produce high-quality recycled raw materials.

Meanwhile, the depolymerization product generated from depolymerization contains, in addition to polyols, compounds such as diamines. However, since such compounds, including diamines, are not separated and the depolymerization product itself is applied to the re-synthesis of polyurethane, it may be difficult to control the quality of the recycled polyurethane produced. Korean Registered Patent No. 10-1447247 (published October 8, 2014) discloses a method for improving the color of recycled polyol and recycled polyurethane produced therefrom, in which the raw material is mixed and heated with a polyhydric alcohol, and then reacted at a reaction temperature of 170 °C to 200 °C by applying an amine-based oligomer, an animal or vegetable oil, and a Bi- or Mo-based catalyst, followed by applying an anti-emulsifier to separate and produce the recycled polyol.

Conventional technologies for chemically decomposing polyurethane by adding a polyhydric alcohol as a solvent for recycling are carried out under high-temperature reaction conditions close to 200 °C. In order to improve the quality (such as color and reactivity) of the recovered recycled polyol, complex purification processes are required, such as continuously supplying nitrogen during the depolymerization process to minimize contact with external air or configuring reaction conditions that can suppress side reactions caused by the introduction of oxides. Therefore, to produce products with consistent quality, high operating costs are required, and there remains considerable room for improvement in terms of energy efficiency.

The present patent aims to overcome the drawbacks of the prior art by applying a hydrophobic co-solvent, which has strong interactions with the hard segment (HS) adjacent to the urethane bond, as a component of the reaction system to lower the activation energy of the depolymerization reaction. Through this, the depolymerization reaction can be induced to proceed rapidly even at a relatively low reaction temperature, while simultaneously providing an environment that can minimize side reactions caused by oxidation during the decomposition process of polyurethane.

In addition, the present patent seeks to present an efficient and novel glycolysis reaction pathway through which a high-quality polyol raw material-easy to recover from the depolymerization of a polymer containing urethane bonds, such as waste polyurethane, and not exhibiting a dark color so as to be applicable to the production of various polyurethanes-can be obtained from the depolymerization of such polymers.

### [Detailed Description of the Invention]

### [Technical Problem]

The present invention aims to provide an effective and economical depolymerization composition for a polymer containing urethane bonds, a depolymerization method for such a polymer, and a method for producing high-quality, high-yield polyol therefrom, wherein the depolymerization is carried out via a glycolysis reaction pathway to improve the performance of the reaction and enable the production of high-quality recycled polyol at a high yield.

### [Technical Solution]

To solve the above problem, the present invention provides a depolymerization composition for a polymer containing a urethane functional group, the composition comprising:
(1) a compound having two or more alcohol functional groups (-OH); and
(2) an aromatic compound containing one or more alkoxy functional groups.

The compound having two or more alcohol functional groups may be one or more selected from the group consisting of ethylene glycol, trimethylene glycol, 1,2-propanediol, tetramethylene glycol, neopentyl glycol, pentamethylene glycol, hexamethylene glycol, decamethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, 2,2-bis(4-β-hydroxyethoxyphenyl)propane, glycerol, pentanetriol, and hexanetriol; and the aromatic compound containing one or more alkoxy functional groups may be one or more selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, ethoxybenzene, and butoxybenzene.

In one embodiment of the present invention, the compound having two or more alcohol functional groups and the aromatic compound containing one or more alkoxy functional groups may be mixed at a weight ratio of 1:20 to 20:1.

The depolymerization composition of the present invention may further comprise a depolymerization catalyst for a polymer containing a urethane functional group, the catalyst being one or more selected from the group consisting of metal catalysts such as alkali hydroxides, alkaline earth hydroxides, alkali acetates, alkaline earth acetates, alkali carbonates, alkali bicarbonates, alkaline earth carbonates, and alkali oxides, or organic compounds of the guanidine or amine type.

In addition, the present invention provides a method for depolymerizing a polymer containing a urethane functional group, the method comprising the step of bringing the polymer containing a urethane functional group into contact with a mixed solvent in which a compound having two or more alcohol functional groups and an aromatic compound having one or more alkoxy functional groups are mixed.

In one embodiment of the depolymerization method of the present invention, the mass of the polymer containing a urethane functional group may be 1 wt% to 200 wt% relative to the mass of the mixed solvent in which the compound having two or more alcohol functional groups and the aromatic compound having one or more alkoxy functional groups are mixed.

In another embodiment of the depolymerization method of the present invention, the mixed solvent may be at a temperature in the range of 100 °C to 170 °C, and the contact between the mixed solvent-comprising a mixture of a compound having two or more alcohol functional groups and an aromatic compound having one or more alkoxy functional groups-and the polymer containing a urethane functional group may be carried out in the presence of one or more catalysts selected from the group consisting of metal catalysts such as alkali hydroxides, alkaline earth hydroxides, alkali acetates, alkaline earth acetates, alkali carbonates, alkali bicarbonates, alkaline earth carbonates, and alkali oxides, or organic compounds of the guanidine or amine type, wherein the total mass of the catalyst may be in the range of 0.0001 to 0.5 times the mass of the polymer containing a urethane functional group.

In another embodiment of the depolymerization method of the present invention, a step of purging with an inert gas prior to or at the initial stage of contact between the mixed solvent-comprising a mixture of a compound having two or more alcohol functional groups and an aromatic compound having one or more alkoxy functional groups-and the polymer containing a urethane functional group may be additionally included.

In another embodiment of the depolymerization method of the present invention, after the step of bringing the mixed solvent-comprising a mixture of a compound having two or more alcohol functional groups and an aromatic compound having one or more alkoxy functional groups-into contact with the polymer containing a urethane functional group, the resulting liquid reaction mixture may be cooled to induce phase separation.

In addition, the present invention provides a method for producing polyol by depolymerizing a polymer containing a urethane functional group, the method comprising the step of bringing a mixed solvent-comprising a mixture of a compound having two or more alcohol functional groups and an aromatic compound having one or more alkoxy functional groups-into contact with the polymer containing a urethane functional group to break the urethane bonds within the polymer and obtain the polyol.

### [Advantageous Effects of the Invention]

According to the present invention, it is possible to depolymerize a polymer containing a urethane functional group and produce high-quality polyol therefrom without using high-temperature, high-pressure reaction conditions.

According to the present invention, by applying, to the depolymerization of a polymer containing urethane bonds, a mixed solvent-comprising a mixture of an aromatic compound having one or more alkoxy functional groups and a compound having two or more alcohol functional groups (-OH)-which is additionally introduced to enhance reaction activity, a polymer containing urethane bonds in a low-density solid form, such as urethane foam, can be more readily dissolved in the reaction solvent, thereby allowing it to be prepared as a liquid-phase depolymerization reactant within a short time (or short cycle). In the depolymerization stage, unlike conventional reactions, the activation energy barrier of the reaction can be lowered, thereby significantly increasing the depolymerization reaction rate.

In addition, the aromatic compound having one or more alkoxy functional groups forms strong intermolecular interactions (π-π interactions and hydrogen bonding) with the portions of the polymer containing urethane functional groups where the urethane bonds are formed. This can more dominantly induce nucleophilic attack by the depolymerization catalyst or the polyhydric alcohol, and, due to its hydrophobicity, can also greatly suppress side reactions. As a result, complete decomposition of polyurethane can be induced within a short reaction time even at a low temperature below the boiling point of the constituent solvent during depolymerization, and, since side reactions are suppressed, a high-quality polyol with almost no discoloration can be obtained as the depolymerization product.

The aromatic compound having one or more alkoxy functional groups according to the present invention has very limited solubility with the compound having two or more alcohol functional groups used as a reactant at temperatures of 100 °C or lower, but it is well miscible with the polyol produced by depolymerization, which is the intended product. Accordingly, when the temperature of the reaction mixture is lowered to 100 °C or below after the depolymerization reaction, most of the unreacted compound having two or more alcohol functional groups becomes concentrated in the hydrophilic aqueous layer, and does not mix with the organic phase composed of a mixture of the aromatic compound having one or more alkoxy functional groups and the polyol product thus existing as a separate phase. From this, the unreacted compound having two or more alcohol functional groups can be easily and simply separated from the reaction product by physical liquid-liquid extraction alone. Furthermore, by removing the co-solvent from the phase-separated organic phase product through relatively simple methods such as evaporation, distillation, or extraction, high-purity polyol can be obtained as the final product. Therefore, the present invention provides the advantage of economically obtaining a transparent recycled polyol product that has been difficult to obtain through conventional recycling methods, and this product can be reused as a polymerization raw material to synthesize a high-quality recycled polymer containing urethane functional groups.

The aromatic compound having one or more alkoxy functional groups used in the present invention can be synthesized on a large scale either naturally or artificially, and most of them are biodegradable in nature within a short cycle, thereby enabling the provision of environmentally friendly operating conditions. Since a polyol raw material that can be used to reproduce materials of the same or similar quality is directly produced through the depolymerization of waste plastics, the present invention can provide an environmentally friendly and economical method for depolymerizing a polymer containing a urethane functional group and producing recycled polyol therefrom, as well as a method for designing a production process for this purpose.

The depolymerization of a polymer containing a urethane functional group according to the present invention can be applied to a simple process design, can proceed at a relatively low temperature, and can yield high-purity and high-yield polyol, thereby providing a very effective and efficient method for the chemical recycling of waste plastics.

In addition, by reverting an already synthesized product back to its raw materials prior to synthesis, the present invention can theoretically enable the infinite reuse of materials, thereby contributing to the realization of a plastic circular economy. Since the depolymerization reaction, product purification, and re-polymerization can be configured together in a continuous manner, it is possible to establish an integrated chemical process capable of producing recycled polymers on a large scale without the need for additional reinvestment or modification of existing polymerization facilities.

### [Brief Description of the Drawings]

**FIG. 1** is a comparison of the FT-IR spectra of the recycled polyol produced according to Example 3 of the present invention, the waste polyurethane (Raw Material 1) used as the raw material for depolymerization, and the virgin polyol (Raw Material 2) initially used for synthesizing said waste polyurethane.
**FIG. 2** is a photograph showing the physical appearance of the recycled polyol products produced according to Examples 1 to 10 and Comparative Examples 2 and 3 of the present invention.

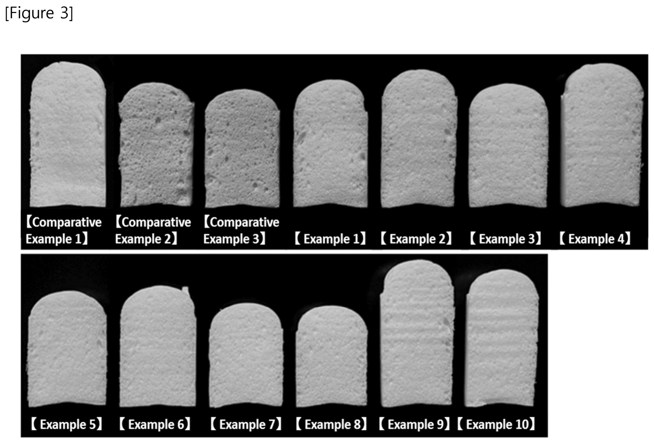

**FIG. 3** is a photograph showing the cross-sectional forms of polyurethane foams manufactured using the recycled polyols produced according to Examples 1 to 10 and Comparative Examples 1 to 3 of the present invention.
**FIG. 4** is a photograph, taken using a stereomicroscope, showing the internal foam layers of polyurethane foams manufactured from recycled polyols produced according to certain Examples and Comparative Examples of the present invention.
**FIG. 5** is a photograph showing the physical appearance of recycled polyol products produced by applying different types of co-solvents in the depolymerization reaction according to Examples 5, 11, and 12 of the present invention.
**FIG. 6** is a photograph showing the cross-sectional forms of polyurethane foams manufactured using the recycled polyols produced according to Examples 5, 11, and 12 of the present invention.

### [Best Mode for Carrying Out the Invention]

Unless otherwise defined, all technical and scientific terms used herein have the same meaning as commonly understood by those skilled in the art to which the present invention pertains. In general, the nomenclature used in this specification is well known and commonly employed in the relevant technical field.

Throughout this specification, when a part is described as "comprising" a certain component, it means that, unless specifically stated otherwise, the description does not exclude the inclusion of other components in addition to the stated component.

The polymer containing a urethane functional group, such as polyurethane, used as the depolymerization raw material in the present invention may have the urethane functional group within its structure as the main bonding group connecting the monomer units constituting the polymer, and may be, for example, flexible polyurethane or rigid polyurethane. In addition, it may include polyethylene, high-density polyethylene, low-density polyethylene, polypropylene, polyethylene terephthalate, or combinations thereof, but is not limited to the aforementioned types of polymers. It may be in the form of a mixture or copolymer with other known polymers, or may contain various types of organic or inorganic foreign substances.

As an example of the polymer containing a urethane functional group, polyurethane is a polymer in the form of a multiblock copolymer containing urethane functional groups, and is composed of hard segments (HS) with a rigid structure that maintain strong cohesion and thermodynamic stability adjacent to the urethane bonds, and soft segments (SS) that form flexible polymer chains to impart elasticity. Such polyurethane may be a polymer having various types, forms, and properties.

The portion constituting the HS of the polyurethane may be formed from various types of diisocyanates, although aromatic diisocyanates are the most common. Representative examples of aromatic diisocyanates that form the HS through the synthesis of polyurethane include monomeric forms such as toluene diisocyanate (TDI), methylene diphenyl diisocyanate (MDI), and m-xylene diisocyanate (MXDI), but polymeric forms, such as polyisocyanates, which are easily soluble in organic phases and thus easy to handle, may also be used. The SS forming the flexible polymer chains of the polyurethane may be in the form of a polyether polyol or a polyester polyol.

The present invention provides a depolymerization composition for a polymer containing a urethane functional group, a depolymerization method for a polymer containing a urethane functional group using said composition, and a method for producing polyol through such depolymerization, the methods being characterized in that a compound having two or more alcohol functional groups is used as a reactant an aromatic compound having one or more alkoxy functional groups is further added as a co-solvent, and the reaction mixture containing the co-solvent is brought into contact with the polymer containing a urethane functional group to break the urethane bonds within the polymer.

The depolymerization composition for a polymer containing a urethane functional group is characterized by comprising: (1) a compound having two or more alcohol functional groups (-OH); and (2) an aromatic compound containing one or more alkoxy functional groups.

The compound having two or more alcohol functional groups may be a glycol having two alcoholic functional groups per molecule, glycerol having three alcoholic functional groups, or a compound having four or more alcoholic functional groups. Examples include dihydric alcohols such as ethylene glycol, trimethylene glycol, 1,2-propanediol, tetramethylene glycol, neopentyl glycol, pentamethylene glycol, hexamethylene glycol, decamethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, and 2,2-bis(4-β-hydroxyethoxyphenyl)propane; trihydric alcohols such as glycerol, pentanetriol, and hexanetriol; polyhydric alcohols containing four or more alcoholic functional groups; or combinations thereof.

The compound having two or more alcohol functional groups corresponds to a hydrophilic solvent and since the catalyst added for depolymerization generally has characteristics close to hydrophilicity, the compound having two or more alcohol functional groups functions not only as a solvent for the polymer containing a urethane functional group but also as a solvent for dissolving the catalyst in the reactant.

The aromatic compound having one or more alkoxy functional groups has at least one aromatic ring, and is an organic compound in which at least one of the hydrogens bonded to the carbon of the hydrocarbon constituting the aromatic ring is substituted with an alkoxy functional group. This compound may be a liquid solvent exhibiting hydrophobicity at the depolymerization reaction temperature.

Examples of the aromatic compound having one or more alkoxy functional groups include one or more compounds selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, ethoxybenzene, and butoxybenzene.

The aromatic compound having one or more alkoxy functional groups forms an unstable thermodynamic phase with the compound having two or more alcohol functional groups at 100 °C or lower, but has high solubility in the polyol generated from the depolymerization, thereby concentrating the resulting polyol at a high concentration in the organic phase layer.

Since the organic phase layer, which contains most of the polyol produced from the depolymerization reaction, forms a distinct phase separation from the hydrophilic layer mainly composed of the compound having two or more alcohol functional groups over a wide temperature range, very simple, stable, and effective separation is possible, and high-yield polyol products can be purified and obtained therefrom.

In addition, the aromatic compound having one or more alkoxy functional groups is a compound capable of strong interactions (π-π interactions and hydrogen bonding) with the urethane bonds and functional groups in the adjacent polymer structure. When introduced into the depolymerization composition for a polymer containing a urethane functional group, it not only rapidly dissolves the polymer containing a urethane functional group but also promotes nucleophilic attack by the catalyst and the compound having two or more alcohol functional groups (diol or polyol) on the polymer containing a urethane functional group. Accordingly, it can accelerate the depolymerization reaction rate and induce depolymerization with excellent selectivity for the polyol product. Furthermore, during the recovery of the converted monomers, the solvents and unreacted materials used in the reaction can be easily recovered and reused through simple physical separation methods such as filtration, distillation, evaporation, or extraction.

Thus, when depolymerizing a polymer containing a urethane functional group using the depolymerization composition according to the present invention, the polymer containing a urethane functional group not only undergoes rapid dissolution even in the absence of a catalyst for depolymerization, but can also be depolymerized at a relatively fast rate even at a low temperature (160 °C or lower) where the rate of side reactions is slow, thereby enabling very effective control of the depolymerization reaction.

One of the features of the present invention is that the depolymerization composition comprises a mixed solvent, serving as the reaction solvent for carrying out depolymerization, the mixed solvent including at least one compound selected from the aromatic compounds having one or more alkoxy functional groups and at least one compound selected from the compounds having two or more alcohol functional groups.

The compound having two or more alcohol functional groups may participate as part or all of the reactant in the depolymerization and, upon cleavage of the urethane bonds, be added to generate polyol corresponding to the depolymerization product. The compound having two or more alcohol functional groups simultaneously serves as a reactant for depolymerization and as a medium that dissolves the polymer containing a urethane functional group to enable the reaction to proceed in the liquid phase. The aromatic compound having one or more alkoxy functional groups does not directly participate as a reactant in the depolymerization reaction but by dissolving the polymer containing a urethane functional group, increases the liquefaction rate of the polymer containing a urethane functional group and, during depolymerization, acts as a co-solvent that facilitates nucleophilic attack by the compound having two or more alcohol functional groups through strong interactions with the urethane bonds, thereby exhibiting the characteristic of improving the depolymerization reaction rate at low temperature.

In the mixed solvent, the aromatic compound having one or more alkoxy functional groups and the compound having two or more alcohol functional groups are present at a weight ratio of 1:20 to 20:1, preferably 1:10 to 10:1, and more preferably 1:2 to 2:1.

The depolymerization composition of the present invention may also further comprise a catalyst for depolymerizing a polymer containing a urethane functional group.

The catalyst may be one capable of improving the depolymerization reaction rate of a polymer containing a urethane functional group, and may be one or more selected from the group consisting of metal catalysts such as alkali hydroxides, alkaline earth hydroxides, alkali acetates, alkaline earth acetates, alkali carbonates, alkali bicarbonates, alkaline earth carbonates, and alkali oxides, or organic compounds of the guanidine or amine type, for breaking the urethane bonds.

In the depolymerization composition, the total mass of the catalyst, when the depolymerization composition is subsequently applied to the depolymerization of a polymer containing a urethane functional group, may be in the range of 0.0001 to 0.5 times, preferably in the range of 0.001 to 0.1 times, the mass of the polymer containing a urethane functional group.

In addition, the present invention provides a method for depolymerizing a polymer containing a urethane functional group.

The depolymerization method according to the present invention may be characterized in that a mixed solvent, comprising a mixture of a compound having two or more alcohol functional groups and an aromatic compound having one or more alkoxy functional groups, is brought into contact with a polymer containing a urethane functional group to break the urethane bonds within the polymer. The types, mixing ratios, and other details of the compound having two or more alcohol functional groups and the aromatic compound having one or more alkoxy functional groups are the same as described above in the section relating to the depolymerization composition, and thus repeated description is omitted.

In the depolymerization method of a polymer containing a urethane functional group according to the present invention, the mass of the polymer raw material initially charged may be adjusted to a ratio of 1 wt% to 200 wt% relative to the mass of the mixed solvent for depolymerization, i.e., the mixed solvent comprising the aromatic compound having one or more alkoxy functional groups and the polyhydric alcohol compound. Charging the polymer raw material within this range enables the formation of a uniform reactant for depolymerization, stable maintenance of the reaction, and securing of uniform product quality, while also improving the productivity and economic efficiency of the depolymerization process for a polymer containing a urethane functional group.

In the depolymerization method of a polymer containing a urethane functional group, the mixed solvent may be heated to a temperature of 100 °C or higher to dissolve the polymer containing a urethane functional group, and the reaction temperature for depolymerization may be the same or higher, and may be in the range of 100 °C to 170 °C, preferably in the range of 140 °C to 165 °C.

In order to improve the depolymerization reaction rate of the polymer containing a urethane functional group in the present invention, the aforementioned catalyst may be added, and the total mass of the catalyst charged for the depolymerization may be in the range of 0.0001 to 0.5 times, preferably 0.001 to 0.1 times, the mass of the polymer containing a urethane functional group.

The catalyst charged for the depolymerization may be added either before or after heating the reaction mixture for depolymerization, and may be introduced directly into the reaction mixture or added after being dissolved in a portion of the solvent.

Since the depolymerization of the polymer containing a urethane functional group and the method for producing polyol proceed at a temperature below the boiling point of the applied reaction solvent, no positive pressure may be generated; however, when a solvent having a relatively low boiling point is used, the depolymerization may be carried out under a low absolute pressure of 1.0 atm to 1.5 atm.

The depolymerization method of a polymer containing a urethane functional group according to the present invention, by using the aromatic compound having an alkoxy functional group as a co-solvent, has the characteristic of significantly suppressing oxidation of the depolymerization product caused by side reactions. However, it may be advantageous for the gaseous phase occupying the volume other than the reactants to contain as little oxygen as possible. Therefore, prior to or at the initial stage of the reaction, the depolymerization may be carried out under conditions in which the system is filled with an inert gas such as helium, argon, or nitrogen through repeated purging, or under conditions with a partial flow of such inert gas.

In the method for depolymerizing a polymer containing a urethane functional group according to the present invention, the depolymerization reaction time may vary depending on the amount and form of the polymer applied. However, when the composition and conditions of the reactants are applied to perform rapid depolymerization, the polymer undergoes predominant decomposition within the initial reaction time of 1 hour due to a very high depolymerization reaction rate, and after 2 hours of reaction, most of the initial urethane bonds are decomposed.

In adjusting the depolymerization reaction time according to the present invention, the depolymerization may be carried out for 1 to 8 hours after the start of the reaction to allow sufficient decomposition of the polymer. However, in order to secure adequate yield, quality, and productivity of the resulting polyol, it may be preferable to conduct the depolymerization for 2 to 4 hours.

In one embodiment of the present invention, the aromatic compound having one or more alkoxy functional groups exhibits very limited solubility with the compound having two or more alcohol functional groups used as a reactant at 100 °C or lower, but is well miscible with the polyol produced from the depolymerization. However, in the temperature range of 100 °C to 170 °C corresponding to the depolymerization reaction conditions, stirring of the reactants is performed in parallel, and thus an emulsion phase having thermodynamic discontinuity may be formed, or the reactants may exist as a single phase. In particular, when most of the urethane bonds are decomposed through depolymerization in the temperature range of 140 °C to 165 °C, the reactants are characterized by existing as a single phase.

In the method for depolymerizing a polymer containing a urethane functional group according to the present invention, unreacted materials and solid foreign substances may be removed from the reaction product after the depolymerization reaction, either before or after the termination of the reaction, by various physical methods such as precipitation, filtration, coagulation, flotation, or pressing, and may be reintroduced into the depolymerization process. In the case of using filtration as the removal method, a filter having fine pores smaller than the particle size of the unreacted polymer may be used, and pressurization or reduced pressure may be applied in parallel to increase the flow rate of the filtrate.

When the liquid reaction mixture obtained as a result of the depolymerization is cooled back down to 100 °C or lower, phase separation occurs, with most of the unreacted compound having two or more alcohol functional groups being concentrated in the hydrophilic solution layer, which can be easily recovered through physical separation alone. The liquid mixed product containing polyol, which is separated into a different phase from the hydrophilic solution layer, can be subjected to relatively simple separation methods such as evaporation, distillation, or extraction to remove the co-solvent, thereby obtaining very high-purity polyol as the final product.

To induce clearer phase separation, one or more polar and non-polar solvents may be further added to the reaction mixture obtained as a result of the depolymerization, and separation may be carried out.

The recycled polyol obtained from the separation process may be used as part or all of the raw material for re-polymerization, and is characterized by being utilizable in the synthesis of new polyurethane materials.

Part or all of the solvent recovered from the separation process may be reused as part of the composition for preparing the mixed solvent required for the depolymerization reaction described above.

In addition, the present invention provides a method for producing polyol by depolymerizing a polymer containing a urethane functional group.

The method for producing polyol is characterized by bringing a reaction mixture comprising a compound having two or more alcohol functional groups and an aromatic compound having one or more alkoxy functional groups into contact with a polymer containing a urethane functional group, thereby breaking the urethane bonds within the polymer and obtaining the polyol.

The compositions and conditions related to the above method are the same as those of the depolymerization method for a polymer containing a urethane functional group, and thus the description thereof is omitted.

Hereinafter, the details of the process of the present invention will be described through Examples, Comparative Examples, and Experimental Examples. These are merely representative illustrations related to the present invention and are not intended to limit the scope of application of the present invention thereto.

### Raw Material 1 (Waste Polyurethane)

Flexible waste polyurethane foam recovered from discarded mattresses was cut and used. The foam was washed with excess ethanol and water, thoroughly dried, then cut and crushed so that one side of the cut surface was 2 mm or less, and the resulting polyurethane chips were prepared as Raw Material 1.

### Raw Material 2 (Virgin Polyol Raw Material)

The polyether polyol (OH value = 56 mgKOH/g, viscosity = 480 cP) used in the production of Raw Material 1 was supplied by the manufacturer and prepared as Raw Material 2 without further purification.

### Raw Material 3 (Polyol Raw Material for Polyurethane Synthesis)

To the polyether polyol of Raw Material 2, based on 100 wt% of the total composition, 0.2 wt% of water, 2 wt% of a catalyst for urethane polymerization, 1.5 wt% of a surfactant, and 2 wt% of a foaming agent were added, respectively, to prepare Raw Material 3 as the polyol for recycled urethane polymerization.

### Raw Material 4 (Diisocyanate Raw Material)

The polymer-type methylene diphenyl diisocyanate (MDI) raw material, modified to a liquid form (NCO value = 33%, viscosity = 17 cP), used in the production of Raw

Material 1, was supplied by the manufacturer and prepared as Raw Material 4 without further purification.

### Comparative Example 1

Polyol of Raw Material 2 was measured at a mass ratio of 1:4 with Raw Material 3, in which additives had been blended, and mixed for about 2 hours using a magnetic stirrer to prepare a mixed polyol raw material for polyurethane synthesis. The rotor speed of a dispersing tool (S25N-18G) connected to a homogenizer (IKA ULTRA-TURRAX) was adjusted to 28,000 rpm, after which 12 g of the prepared mixed polyol raw material and 3 g of isocyanate of Raw Material 4 were combined and subjected to the homogenizer to induce uniform mixing for 6 seconds. The mixture was then quickly transferred into a polypropylene container with an inner diameter of 33 mm and a volume of 50 ml, and the formation process of the polyurethane foam was observed. After exposing the foam to ambient air for 24 hours, the manufactured foam was cut along the longitudinal growth axis so that the cut surfaces were symmetrical and each cut foam piece had the same shape. The cut surfaces were examined under a stereomicroscope to observe the structure of the bubble layers formed during the polyurethane foam production process.

### Comparative Example 2

### (a) Depolymerization of Polyurethane (Polymer Containing Urethane Functional Group)

Approximately 20.0 g of polyurethane foam from Raw Material 1 was used as the polymer raw material for depolymerization. The weighed polyurethane foam was charged into a 500 mL three-neck flask, to which only 18 g of anhydrous ethylene glycol-without using a co-solvent-was measured and added to prepare the initial reactant for depolymerization. A condenser equipped with an internal thermocouple for temperature measurement and a septum made of Teflon-silicone for liquid sample collection were connected to the side necks of the three-neck flask containing the reactant via bushing-type adapters. A Teflon seal connected to the central neck of the flask allowed a stirring shaft, driven by an external overhead stirrer, to connect to an impeller inside the reactor for mixing, thereby isolating the reactor from ambient air to prepare it for depolymerization. The flask was placed in a thermostatic bath filled with high-temperature methyl phenyl silicone oil, which was maintained at a constant temperature using a PID temperature controller, and the mixture was stirred at 250 rpm until the internal temperature of the reactor reached 200 °C. When the internal temperature stabilized, 0.1 g of sodium hydroxide (NaOH) was dissolved in 2 g of prepared ethylene glycol, and the resulting catalyst solution was introduced to initiate the reaction. Attenuated Total Reflection Fourier-Transform Infrared Spectroscopy (ATR/FT-IR; Bruker ALPHA II) was performed on the polyurethane, the initial polyol used for polyurethane synthesis, and the regenerated polyol obtained by sampling part of the reaction mixture during depolymerization. The resulting spectra were compared to determine whether depolymerization had been completed.

### (b) Recovery of Reaction Solvent

After the reaction was carried out at 200 °C for 4 hours, the reactor was removed from the oil thermostatic bath to terminate the reaction. When the temperature had dropped below 100 °C and the phase boundary became clearly visible, the unreacted ethylene glycol in the lower phase, along with a large amount of catalyst, was recovered using a separatory funnel. The organic compounds in the upper phase were transferred into a 100 mL evaporation flask with a pre-measured tare weight and then weighed.

### (c) Recovery of Polyol Product

The upper-phase mixture obtained above was placed in the 100 mL evaporation flask and mounted on a rotary evaporator. The flask was rotated at 150 rpm in continuous contact with a thermostatic water bath maintained at 65 °C under reduced pressure (10 torr) for approximately 1 hour to completely remove the distillate. The remaining highly viscous, opaque black liquid (13.18 g) in the rotary evaporator was collected as the polyol product.

### (d) Characterization of the Polyol Product

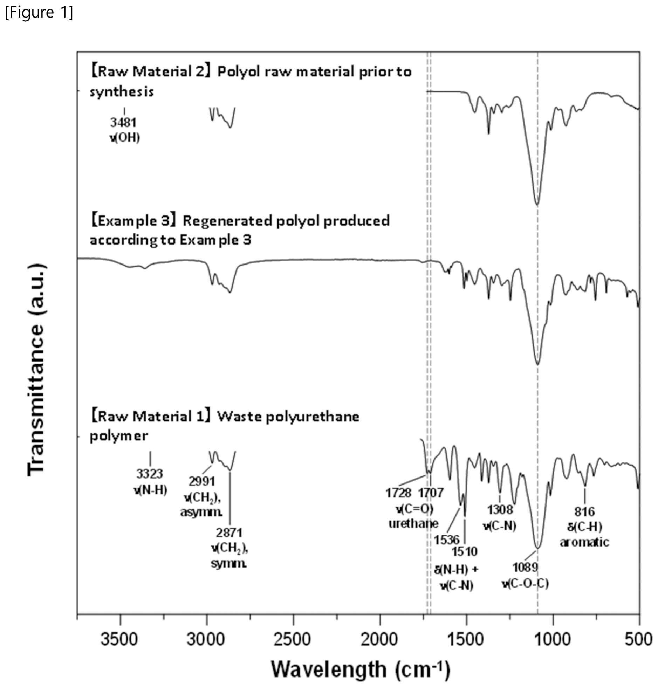

In order to analyze the bonding structure of the functional groups of the recycled polyol (product obtained from the depolymerization of Raw Material 1) ultimately produced according to the depolymerization process described in (a) above, a portion was taken as an ATR sample and subjected to FT-IR analysis, the results of which are shown in FIG. 1.

Meanwhile, the structural characteristics of the initial polyol (Raw Material 2) used for polyurethane synthesis and the recycled polyol produced from the depolymerization were compared using ^1H-NMR. The ^1H-NMR samples were prepared by diluting the polyols in N,N-dimethylformamide (DMF)-d₇ to a final polyol concentration of 6.25%. The viscosities of the initial polyol (Raw Material 2) and the recycled polyol (product obtained from the depolymerization of Raw Material 1) were measured using an automatic rotational viscometer (IKA ROTAVISC lo-vi with VOLS-1). A portion of the polyol product obtained from the depolymerization was used to measure the acid value and hydroxyl value of the produced polyol in accordance with the procedures and methods specified in ASTM D4662-20 and ASTM D4274-21, respectively.

In addition, the color characteristics of the recycled polyol thus produced were measured using a spectrophotometer (Manufacturer: Konica Minolta, Model: CM-3600A).

### (e) Synthesis of Recycled Polyurethane

Except that the recycled polyol produced through the depolymerization process described in (a) above was used as the polyol raw material in place of the polyol of Raw Material 2, polyurethane foam was manufactured and analyzed in the same manner and procedure as in Comparative Example 1.

### Comparative Example 3

In the step **(a) Depolymerization of Polyurethane** of Comparative Example 2, after preparing the initial reaction mixture for depolymerization, a step of purging the reactor by flowing nitrogen at a flow rate of 100 sccm for 45 minutes to remove oxygen inside the reactor prior to isolating it from the external atmosphere was additionally performed. Except for this, the depolymerization reaction was carried out in the same manner and procedure as in Comparative Example 2. Through the same purification method as in step (c) of Comparative Example 2, about 13.20 g of an opaque, dark brown polyol was finally obtained. The properties of the polyol produced were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 1

In the step **(a) Depolymerization of Polyurethane** of Comparative Example 2, about 20.0 g of the polyurethane foam of Raw Material 1, 18 g of anhydrous ethylene glycol, and 14 g of anisole were precisely measured to prepare the initial reaction mixture for depolymerization; the amount of sodium hydroxide (NaOH) used was doubled to 0.2 g; and the depolymerization was conducted under a lower-temperature reaction condition (160 °C). Except for these differences, the depolymerization reaction was carried out in the same manner and procedure as in Comparative Example 2. The same method as in step (c) of Comparative Example 2 was applied, except that the co-solvent was also removed, thereby finally obtaining about 14.69 g of a pale yellow, translucent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 2

In the depolymerization step of Comparative Example 3, about 20.0 g of the polyurethane foam of Raw Material 1, 18 g of anhydrous ethylene glycol, and 14 g of anisole were precisely measured to prepare the initial reaction mixture for depolymerization; thereafter, as in Comparative Example 3, before isolating the reaction system from the external atmosphere, nitrogen was flowed at a rate of 100 sccm for 45 minutes to remove oxygen inside the reactor; and the depolymerization was conducted under a lower-temperature reaction condition (160 °C). Except for these differences, the depolymerization reaction was carried out in the same manner and procedure as in Comparative Example 3. The same method as in step (c) of Comparative Example 2 was applied, except that the co-solvent was also removed, thereby finally obtaining about 14.55 g of a pale yellow, transparent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 3

Except that a catalyst solution prepared by dissolving 0.1 g of potassium hydroxide (KOH) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 15.39 g of a pale yellow, transparent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 4

Except that a catalyst solution prepared by dissolving 0.1 g of triazabicyclodecene (TBD) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 14.96 g of a pale yellow, transparent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 5

Except that a catalyst solution prepared by dissolving 0.1 g of potassium carbonate (K₂CO₃) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 14.50 g of a pale yellow, transparent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 6

Except that a catalyst solution prepared by dissolving 0.1 g of potassium bicarbonate (KHCO₃) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 15.54 g of an orange, transparent polyol. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 7

Except that a catalyst solution prepared by dissolving 0.1 g of potassium acetate (CH₃COOK) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 7.64 g of a transparent polyol having a slightly dark yellow color. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 8

Except that a catalyst solution prepared by dissolving 0.2 g of potassium acetate (CH₃COOK) in 2 g of ethylene glycol was used in place of sodium hydroxide (NaOH), the depolymerization reaction was carried out in the same manner and procedure as in Example 2. The same method as in step (c) of Comparative Example 2 was applied, except that the co-solvent was also removed, thereby finally obtaining about 14.94 g of a transparent polyol having a pale orange color. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 9

Except that, in the "(a) Depolymerization of Polyurethane" step, about 20.0 g of polyurethane foam of Raw Material 1 was prepared as the initial reaction material for depolymerization by measuring 18 g of diethylene glycol and 14 g of anisole as the reaction solvent instead of ethylene glycol, and that the catalyst solution for initiating the reaction was prepared by adding 0.1 g of potassium hydroxide (KOH) to 2 g of diethylene glycol instead of ethylene glycol, the depolymerization reaction was carried out in the same manner and procedure as in Example 2. The same method as in step (c) of Comparative Example 2 was applied, except that the co-solvent was also removed, thereby finally obtaining about 15.22 g of a transparent polyol having a dark brown color. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 10

Except that, in the "(a) Depolymerization of Polyurethane" step, about 20.0 g of polyurethane foam of Raw Material 1 was prepared as the initial reaction material for depolymerization by measuring 18 g of glycerol and 14 g of anisole as the reaction solvent instead of ethylene glycol, and that the catalyst solution for initiating the reaction was prepared by adding 0.1 g of potassium hydroxide (KOH) to 2 g of glycerol instead of ethylene glycol, the depolymerization reaction was carried out in the same manner and procedure as in Example 2. The same method as in step (c) of Comparative Example 2 was applied, except that the co-solvent was also removed, thereby finally obtaining about 15.82 g of an opaque polyol having a pale yellow color. The properties of the polyol thus prepared were analyzed according to the same method and procedure as in step (d) of Comparative Example 2. In addition, except that the recycled polyol produced through the depolymerization process was used as the polyol raw material in place of the polyol of Raw Material 2, polyurethane foam was manufactured and its properties were observed in the same manner and procedure as in step (e) of Comparative Example 2.

### Example 11

Except that, in the "(a) Depolymerization of Polyurethane" step, about 20.0 g of polyurethane foam of Raw Material 1 was prepared as the initial reaction material for depolymerization by measuring and adding 14 g of ethoxybenzene as the co-solvent instead of anisole, and that the catalyst solution for initiating the reaction was prepared by adding 0.1 g of potassium carbonate (K₂CO₃) to 2 g of ethylene glycol instead of sodium hydroxide (NaOH), the depolymerization reaction was carried out in the same manner and procedure as in Example 2.

In the purification process of the polyol, the co-solvent was removed according to the solvent extraction method to obtain the polyol. Specifically, 50 mL of n-hexane was added to the reaction mixture, followed by stirring at room temperature for 24 hours to remove the co-solvent dissolved in n-hexane. Then, 50 mL of toluene was added to induce phase separation of the depolymerization reaction mixture. The organic phase, mainly composed of toluene and polyol, was transferred into a 100 mL evaporation flask of known empty weight and then measured. The same method as in Comparative Example 2 (c) was applied, except that toluene was also removed through the evaporation process, thereby finally obtaining about 13.80 g of an opaque polyol having a dark yellow color. The properties of the polyol thus prepared were analyzed in the same manner and procedure as in Comparative Example 2 (d). Furthermore, except that the regenerated polyol prepared through the above depolymerization process was applied as the raw material for polyurethane synthesis instead of the polyol of Raw Material 2, polyurethane foam was manufactured and its properties were observed in the same manner and procedure as in Comparative Example 2 (e).

### Example 12

In the "(a) Depolymerization of Polyurethane" step, about 20.0 g of polyurethane foam of Raw Material 1 was measured, and, as a co-solvent, 14 g of 1,4-dimethoxybenzene was measured and added in place of ethoxybenzene to prepare the initial reaction mixture for depolymerization. Except for this change, the depolymerization reaction was carried out in the same manner and procedure as in Example 11. The same method as in Comparative Example 2 (c) was applied, except that the solvent was completely removed through the evaporation process, thereby finally obtaining about 14.15 g of an opaque polyol having a dark yellow color. The regenerated polyol product was obtained in the same manner and procedure as in Example 11, and the properties of the product prepared using this polyol were analyzed.

**[Table 1]**

| **Classificatio n** | Catalyst / Usage Amount (per unit mass of waste PU) | Reactio n Temp. (°0) | Reactio n Solvent * | Initial Nitroge n Chargin g | Mass of Recovere d Polyol (g) | Yellowness Index | | Viscosit y (25°C cP) | OH value (mg KOH/g) | Acid value (mg KOH/g) |
|---|---|---|---|---|---|---|---|---|---|---|
| | | | | | | **AST M E313 -73** | **ASTM D1925** | | | |
| Raw Material 2 | - | - | - | - | - | **0.0** | **0.4** | **480** | 59 | 0.03 |
| Comparative Ex. 2 | NaOH / 0.005 | 200 | EG | × | **13.18** | 99.2 | **283** | **1968** | 310 | 0.11 |
| Comparative Ex. 3 | NaOH / 0.005 | 200 | EG | ○ | **13.20** | 99.3 | **280** | **1971** | 313 | 0.15 |
| Example 1 | NaOH / 0.010 | 160 | EG | × | **14.69** | 92.3 | **129** | **711** | 199 | 0.10 |
| Example 2 | NaOH / 0.005 | 160 | EG | ○ | **14.55** | 87.6 | **111** | **1275** | 280 | 0.09 |
| Example 3 | KOH / 0.005 | 160 | EG | ○ | **15.39** | 84.8 | **107** | **696** | 213 | 0.09 |
| Example 4 | TBD / 0.005 | 160 | EG | ○ | **14.96** | 81.9 | **103** | **709** | 204 | 0.05 |
| Example 5 | K₂CO₃ / 0.005 | 160 | EG | ○ | **14.50** | 78.2 | **94** | **727** | 226 | 0.02 |
| Example 6 | KHCO₃ / 0.005 | 160 | EG | ○ | **15.54** | 85.5 | **108** | **701** | 228 | 0.05 |
| Example 7 | KOAc / 0.005 | 160 | EG | ○ | 7.14 | 84.3 | **106** | **762** | 255 | 0.08 |
| Example 8 | KOAc / 0.010 | 160 | EG | ○ | 14.94 | 80.2 | **99** | **717** | 231 | 0.05 |
| Example 9 | KOH / 0.005 | 160 | DEG | ○ | 15.22 | 96.2 | **146** | **514** | 191 | 0.09 |
| Example 10 | KOH / 0.005 | 160 | Gly | ○ | 15.82 | 84.2 | **104** | **1090** | 139 | 0.14 |

| | | | | | | | | | | |
|---|---|---|---|---|---|---|---|---|---|---|
| *EG: ethylene glycol, DEG: Diethylene glycol, Gly: Glycerol | | | | | | | | | | |

Table 1 compares the reaction conditions for producing regenerated polyols according to the depolymerization method of the present invention and the conventional known high-temperature depolymerization method, as well as the yields and properties of the polyols produced therefrom. It was observed that the predominant depolymerization reaction proceeded within 2 hours, resulting in the decomposition of most of the polyurethane. However, the reaction mixture was exposed to the depolymerization conditions for 4 hours to induce sufficient depolymerization, and the regenerated polyol product was then obtained through a purification process.

By comparing the final spectrum of the regenerated polyol obtained with the spectra of the polyurethane and the initial polyol raw material, and by observing changes in characteristic peaks, information was obtained on the structural changes of the polymer compound resulting from the depolymerization of polyurethane.

Figure 1 shows the spectra of waste polyurethane (raw material 1), the initial polyol (raw material 2) used for its synthesis, and the regenerated polyol obtained from the depolymerization in Example 3, measured using ATR/FT-IR.

In the spectrum of polyurethane (raw material 1), the absorption peaks observed at wavenumbers 1707 cm⁻¹ and 1728 cm⁻¹ correspond to the characteristic peaks of the carbonyl (C=O) group within the urethane bond. In intermediate products in which partial decomposition occurred during the depolymerization process, attenuation of these peaks was observed, while in the spectrum of the regenerated polyol obtained from complete depolymerization, the characteristic peaks representing the urethane bond were not detected. From these changes in characteristic peaks, it was possible to determine whether the depolymerization of waste polyurethane had been completed.

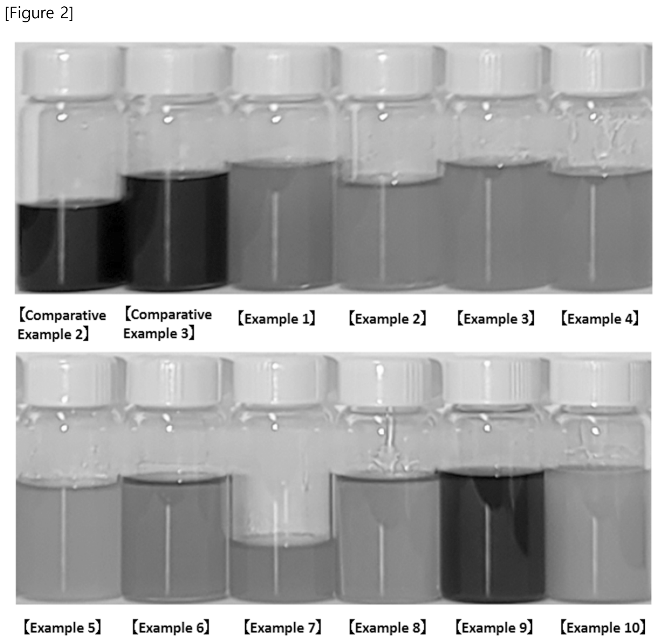

The regenerated polyol samples shown in Table 1 are respectively compared in the photographs of Figure 2. In the case of polyols obtained through high-temperature depolymerization without the use of a co-solvent (Comparative Examples 2 and 3), they exhibited a dark brown or black appearance. However, according to the embodiments of the present invention, when waste polyurethane was decomposed through co-solvent-based low-temperature depolymerization, the regenerated polyol obtained as the final product of the depolymerization reaction and purification process was mostly transparent and light in color. Although not shown in Table 1, when all depolymerization reaction conditions, including temperature (160°C or below), were applied identically but without using a co-solvent or by using aromatic compounds without an alkoxy functional group-such as toluene or p-xylene-instead of the co-solvent according to the present invention, depolymerization of polyurethane scarcely proceeded.

When ethylene glycol is added as a reaction solvent and depolymerization is carried out at high temperature in the presence of a catalyst, the depolymerization proceeds at a relatively fast rate if a high thermal energy supply is provided. However, during the depolymerization process, side reactions such as oxidation-induced degradation or hydrolysis can also proceed rapidly at the same time. These side reactions are known to cause discoloration by producing functional groups such as chromophores and auxochromes. In particular, in polymers where urethane bonds are formed using isocyanates with aromatic structures such as TDI or MDI as raw materials, it is known that discoloration becomes more pronounced when side reactions caused by degradation or hydrolysis occur, because the functional groups responsible for discoloration have a greater effect on the electronic structure of the aromatic ring.

However, according to the present invention, when an aromatic compound having an alkoxy group is used as a co-solvent, it not only significantly lowers the activation energy for the decomposition of urethane bonds by forming strong intermolecular interactions such as hydrogen bonding and π-π interactions, but is also expected to greatly reduce the diffusion rate of oxygen or moisture-which promote discoloration during the decomposition process-by allowing the hydrophobic co-solvent to be present at a high density near the urethane functional groups. As a result, the glycol transesterification reaction can proceed more selectively simultaneously with the decomposition of the urethane functional groups, enabling the production of high-quality depolymerization products.

Since the depolymerization process of polyurethane is a heating reaction process in which decomposition occurs through exposure to high temperature, it may be advantageous to remove oxygen-which promotes side reactions-at the initial stage in order to control the extent of discoloration, and it may also be beneficial to use a catalyst that can enhance the rate of the transesterification reaction.

According to the results of Comparative Examples 2 and 3, it can be seen that in depolymerization reactions carried out at very high temperatures, it is not easy to control side reactions that cause discoloration. In contrast, in the case of regenerated polyols produced in Examples 1 and 2, where depolymerization was performed using a co-solvent, it was found that increasing the amount of catalyst or carrying out the reaction under an initial vacuum atmosphere to control the rate and selectivity of the transesterification reaction led to some differences in the yield and quality of the final polyol obtained.

When the amount of catalyst was increased (Example 1), the waste polyurethane was almost completely decomposed, resulting in a very high recovery yield close to the mass of the polyol used in the initial polymer synthesis (about 75% of the polyurethane, 15 g), and exhibiting low viscosity. However, since depolymerization was carried out without removing oxygen at the initial stage of the reaction, greater yellowing occurred in the final polyol product obtained.

Examples 2 to 7 illustrate a comparison of the results obtained when depolymerization was carried out using waste polyurethane as the raw material, while keeping the mass of catalyst added per unit mass of waste polyurethane constant, and employing as the catalyst an alkali metal hydroxide, an alkali metal carbonate, an alkali metal bicarbonate, an alkali metal acetate, or an organic compound of the guanidine type.

Except for the case where potassium acetate was used as the catalyst, polyols were obtained at very high yields, and unlike in Comparative Examples 2 and 3, the color of the obtained polyols was uniformly transparent yellow. The OH value (mg KOH/g) was in the range of 200 to 300, and the polyols exhibited low viscosity.

Meanwhile, in the case of using potassium acetate as the catalyst, it was found that a depolymerization reaction carried out with an increased amount of catalyst (Example 8) achieved depolymerization performance similar to the others and yielded high-quality regenerated polyol.

When polyhydric alcohols such as diethylene glycol or glycerol were used as the reaction solvent instead of ethylene glycol and applied to the co-solvent-based low-temperature depolymerization according to the present invention (Examples 9 and 10), most of the polyols obtainable from waste polyurethane could be recovered; however, regenerated polyol products of a different form, showing some differences in physical properties (such as color and viscosity) compared to the initial polyol, were obtained.

Figure 2 shows cross-sectional views of polyurethane foams synthesized using different polyol raw materials. Comparative Example 1 was prepared using the initial (virgin) polyol raw material (raw material 2); Comparative Examples 2 and 3 were prepared using regenerated polyols produced by high-temperature depolymerization; and Examples 1 to 10 were prepared using regenerated polyols obtained from the co-solvent-based depolymerization according to the present invention.

Comparative Examples 2 and 3, which used regenerated polyols obtained from high-temperature depolymerization, not only exhibited low reactivity but also formed very coarse and irregular foam cell layers during the foaming process. In contrast, they were found to have a dark color compared to the polyurethane foams synthesized from regenerated polyols obtained in the Examples.

In contrast, the polyurethane foams synthesized using regenerated polyols produced according to the present invention exhibited relatively uniform foam cell formation, and in terms of reactivity and foam color, many of them were difficult to distinguish from those produced using the initial (virgin) polyol raw material.

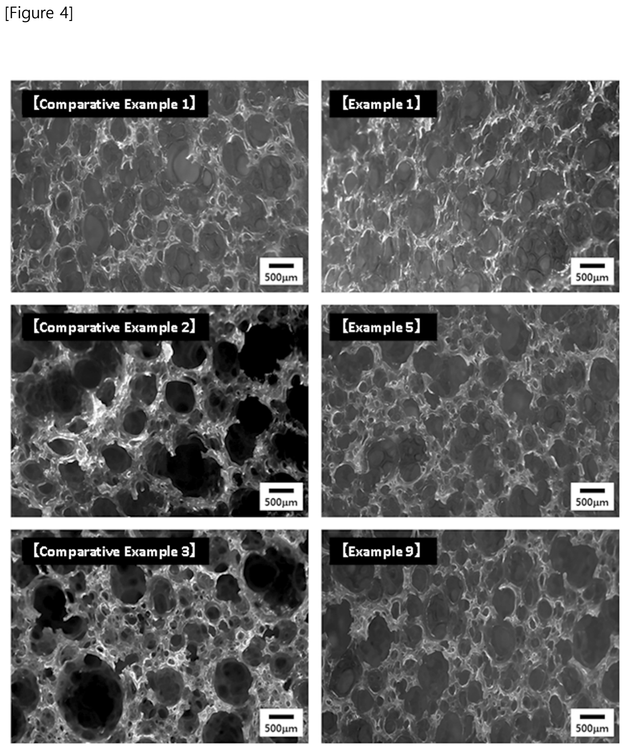

Figure 4 shows a stereomicroscopic observation of local regions of the foam cell layers formed inside some polyurethane foam samples. The polyurethanes synthesized using regenerated polyols produced according to the present invention (Examples 1, 5, and 9) formed relatively uniform foam cell layers, and shapes similar to those observed in the polyurethane prepared using the initial (virgin) polyol raw material (Comparative Example 1) were observed. In contrast, in the polyurethane foams obtained by depolymerization at high temperature without using a co-solvent (Comparative Examples 2 and 3), it can be seen that uniform foam cell layers were not formed.

**[Table 2]**

| Classification | Type and Amount of Co-solvent (per unit mass of waste PU) | Mass of Recovered Polyol (g) | Yellowness Index | | Viscosity (25°CcP) | OH value (mg KOH/g) | Acid value (mg KOH/g) |
|---|---|---|---|---|---|---|---|
| | | | **ASTM E313-73** | **ASTM D1925** | | | |
| Raw material 2 | - | - | **0.0** | **0.4** | **480** | 59 | 0.03 |
| Example 5 | Anisole / 0.005 | **14.50** | 78.2 | **94** | **727** | 226 | 0.02 |
| | | | | | | | |
| Example 11 | EB* / 0.005 | 15.82 | 93.6 | **135** | **1823** | 170 | 0.18 |
| Example 12 | 1,4-DMB** / 0.005 | 15.22 | 94.1 | **138** | **2013** | 297 | 0.09 |

| | | | | | | | |
|---|---|---|---|---|---|---|---|
| [Depolymerization Conditions] Catalyst and amount (per 1 g of waste PU): K₂CO₃ 0.005 g Reaction temperature: 160°C Reaction solvent (per 1 g of waste PU): EG 1 g * EB: ethoxy benzene ** DMB: dimethoxy benzene | | | | | | | |

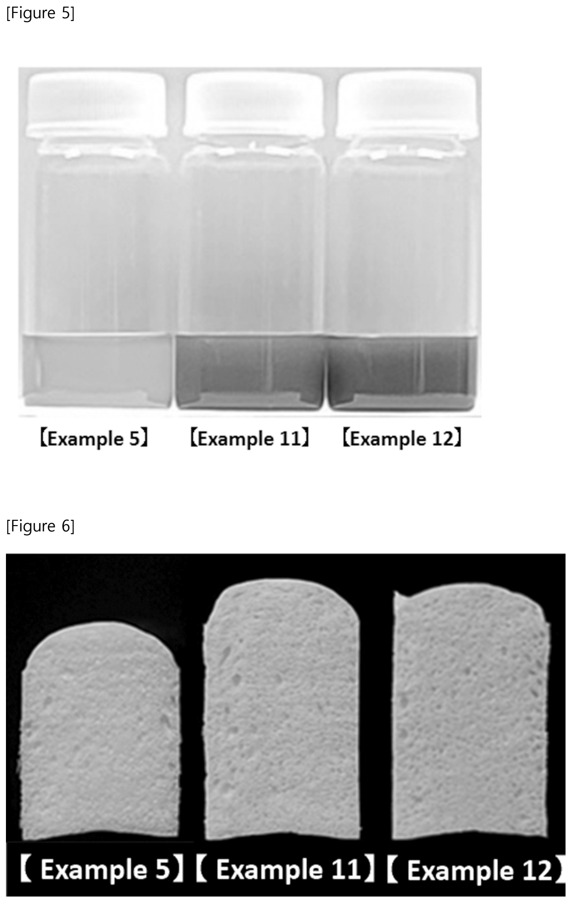

Table 2 shows the yield and properties of the polyols obtained by varying the type of co-solvent used. Photographs of the regenerated polyol samples obtained are shown in Figure 5 for comparison.

Examples 11 and 12 are cases where a compound having a longer alkoxy group or multiple alkoxy groups bonded to an aromatic ring was used as the co-solvent. Similar to the previously conducted examples, complete decomposition of the waste polyurethane occurred within 4 hours through low-temperature depolymerization, and the regenerated polyol produced therefrom was recovered in a mass close to the maximum yield (approximately 15 g of polyol used in the polyurethane synthesis).

Meanwhile, the polyols obtained in Examples 11 and 12 were somewhat darker in color and had higher viscosity compared to the polyol product obtained in Example 5, where anisole was used for depolymerization.

Figure 6 compares the cross-sections of polyurethane foams synthesized using the polyols obtained from Examples 11 and 12 with those obtained from Example 5. Although somewhat coarse foam cell layers were observed during the foaming process, polyurethane foams of improved quality were produced compared to those made using the polyols obtained at high temperature in Comparative Examples 2 and 3.

As described above, certain portions of the present invention have been described in detail; however, to those skilled in the art, such specific descriptions are merely preferred embodiments, and it will be apparent that the scope of the present invention is not limited thereby. Accordingly, the substantive scope of the present invention shall be defined by the appended claims and their equivalents.

## Claims

1. A composition for depolymerizing a polymer containing a urethane functional group,
wherein the composition comprises:
(1) a compound having two or more alcohol functional groups (-OH); and
(2) an aromatic compound containing one or more alkoxy functional groups; wherein the composition is **characterized by** the above.

2. The composition for depolymerizing a polymer containing a urethane functional group according to claim 1,
wherein the compound having two or more alcohol functional groups is one or more selected from the group consisting of ethylene glycol, trimethylene glycol, 1,2-propanediol, tetramethylene glycol, neopentyl glycol, pentamethylene glycol, hexamethylene glycol, decamethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, 2,2-bis(4-β-hydroxyethoxyphenyl)propane, glycerol, pentanetriol, and hexanetriol.

3. The composition for depolymerizing a polymer containing a urethane functional group according to claim 1,
wherein the aromatic compound containing one or more alkoxy functional groups is one or more selected from the group consisting of methoxybenzene, 1,2-dimethoxybenzene, 1,3-dimethoxybenzene, 1,4-dimethoxybenzene, 1,2,3-trimethoxybenzene, 1,2,4-trimethoxybenzene, 1,3,5-trimethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, ethoxybenzene, and butoxybenzene.

4. The composition for depolymerizing a polymer containing a urethane functional group according to claim 1,
wherein the compound having two or more alcohol functional groups and the aromatic compound containing one or more alkoxy functional groups are mixed at a weight ratio of 1:20 to 20:1.

5. The composition for depolymerizing a polymer containing a urethane functional group according to claim 1,
wherein the depolymerization composition further comprises a polymer depolymerization catalyst containing a urethane functional group, the catalyst being one or more selected from the group consisting of metal catalysts such as alkali hydroxides, alkaline earth hydroxides, alkali acetates, alkaline earth acetates, alkali carbonates, alkali bicarbonates, alkaline earth carbonates, and alkali oxides, or organic compounds of the guanidine type or amine type.

6. A method for depolymerizing a polymer containing a urethane functional group, comprising a step of bringing a mixed solvent-comprising a compound having two or more alcohol functional groups and an aromatic compound having one or more alkoxy functional groups-into contact with the polymer containing a urethane functional group.

7. The method for depolymerizing a polymer containing a urethane functional group according to claim 6,
wherein the mass of the polymer containing a urethane functional group corresponds to 1 wt% to 200 wt% of the mass of the mixed solvent comprising the compound having two or more alcohol functional groups and the aromatic compound having one or more alkoxy functional groups.

8. The method for depolymerizing a polymer containing a urethane functional group according to claim 6,
wherein the mixed solvent is in a temperature range of 100°C to 170°C.

9. The method for depolymerizing a polymer containing a urethane functional group according to claim 6,
wherein the contact between the mixed solvent-comprising the compound having two or more alcohol functional groups and the aromatic compound having one or more alkoxy functional groups-and the polymer containing a urethane functional group is carried out in the presence of one or more catalysts selected from the group consisting of metal catalysts such as alkali hydroxides, alkaline earth hydroxides, alkali acetates, alkaline earth acetates, alkali carbonates, alkali bicarbonates, alkaline earth carbonates, and alkali oxides, or organic compounds of the guanidine type or amine type.

10. The method for depolymerizing a polymer containing a urethane functional group according to claim 9,
wherein the total mass of the catalyst is in the range of 0.0001 to 0.5 times the mass of the polymer containing a urethane functional group.

11. The method for depolymerizing a polymer containing a urethane functional group according to claim 6,
wherein purging with an inert gas is carried out prior to or at the initial stage of contact between the mixed solvent-comprising the compound having two or more alcohol functional groups and the aromatic compound having one or more alkoxy functional groups-and the polymer containing a urethane functional group.

12. The method for depolymerizing a polymer containing a urethane functional group according to claim 6,
wherein, after the contact step, the resulting liquid reaction mixture is cooled to induce phase separation.

13. A method for producing a polyol by depolymerizing a polymer containing a urethane functional group,
comprising the step of bringing a mixed solvent-comprising a compound having two or more alcohol functional groups and an aromatic compound having one or more alkoxy functional groups-into contact with the polymer containing a urethane functional group to decompose the urethane bonds in the polymer and obtain a polyol.
